# EUROPEAN PATENT APPLICATION

(11) **EP 4 792 733 A1**
(43) Date of publication of application: **19.08.2026**
(21) Application number: 25157501.5
(22) Date of filing: 12.02.2025
(51) Int. Cl.: A61B 5/00, A61B 5/11

(54) **MONITORING AND VISUALIZING GAIT OF A USER OF AN ASSISTIVE DEVICE**

(71) Applicant: Blatchford Products Limited, Basingstoke, Hampshire RG24 8PZ (GB)
(72) Inventor: Odeyale, Akinkunmi, Northampton, NN3 5ET (GB); Woodhead, Robert Paul, Warwick, CV34 5NS (GB)
(74) Representative: Hepworth Browne

(57) **Abstract**

Embodiments disclosed herein provide a mobile device (103) for monitoring and visualizing a gait of a user (101). The mobile device (103) includes a camera (204), an I/O interface (202), a memory (203) comprising an application (104) and a processor (201) connected to the memory. The processor (201) is configured to start a gait session to receive a measurement data from plurality of sensors of the user (101) wearing an assistive device (102), the gait session initializes the measurement data reception from the plurality of sensors. Further, the processor (201) activates the camera to record a video data of the user (101) concurrently receiving the measurement data from the plurality of sensors during the gait session. The processor (201) applies timestamps to frames of the video data of the user (101) with respect to the measurement data based on the initialization signal to obtain time-synchronized gait visualization data (301).

## Description

### FIELD OF INVENTION

The present disclosure relates to medical devices and, more particularly, to monitoring and visualizing gait of a user wearing an assistive device in prosthetics.

### BACKGROUND

Prosthetics are essential medical devices designed to replace missing body parts, such as fingers, hands, arms, and legs. They play a crucial role in improving the quality of life for individuals who have lost limbs due to injury, congenital conditions, or diseases such as diabetes, dysvascular disease, or tumors. Despite their importance, current prosthetic devices face several challenges that impact their functionality and user experience.

One of the main issues with existing limb prostheses is the significant energy expenditure required for their use compared to that of able-bodied individuals. This energy discrepancy arises from substantial energy losses during the operation of prosthetic limbs, which can lead to user fatigue and reduced mobility. As a result, there is a pressing need to develop active prosthetics that incorporate ergonomic considerations to enhance the quality of life for amputation patients.

In addition to prosthetics, gait analysis is a critical tool used to evaluate an individual's walking pattern to identify abnormalities and areas for improvement. This analysis is particularly important in diagnosing and treating disorders with motor symptoms, especially in neurological diseases where continuous monitoring and treatment adjustments are necessary. Neurological disorders often lead to progressive gait abnormalities and an increased risk of falls, necessitating personalized treatment plans. However, relying solely on clinical observation may not accurately detect underlying issues, highlighting the need for supplementary systems to estimate gait metrics.

Current gait analysis systems face several limitations. They may lack comprehensive data acquisition capabilities, robust visualization tools, or the ability to effectively correlate gait patterns with clinical outcomes. Furthermore, the high cost and complexity of many of these solutions restrict their widespread adoption in clinical environments. Consequently, there is a demand for pervasive and objective gait assessment systems that can be easily deployed outside of hospital settings. These systems should complement existing standards and improve accessibility for patients, enabling healthcare professionals to better understand patient-specific needs, optimize treatment plans, and assess intervention effectiveness.

By providing clear and intuitive visual representations of gait data, these assessment systems can empower clinicians to make more informed decisions, ultimately enhancing patient care. Addressing the aforementioned disadvantages and shortcomings, or at least providing a useful alternative, is of paramount importance in advancing the fields of prosthetics and gait analysis.

### SUMMARY

Embodiments disclosed herein provide a mobile device for monitoring and visualizing a gait of a user. The mobile device includes a camera, an I/O interface, a memory comprising an application, and a processor connected to the memory. The processor is configured to display a user interface via the application; the user interface allows the user to connect to an assistive device worn by the user. The processor establishes a wireless connection with the assistive device via a short-range communication protocol, the assistive device including a plurality of sensors produces measurement data indicating movements of the user. The processor is configured to start a gait session to receive the measurement data from the plurality of sensors upon successful establishment of the wireless connection link; the gait session initializes the measurement data reception from the plurality of sensors. The processor of the mobile device receives an initialization signal from the assistive device confirming readiness of the plurality of sensors to transmit the measurement data. Further, the processor activates the camera to record video data of the user wearing the assistive device concurrently after receiving the initialization signal; the video data is recorded while receiving the measurement data from the plurality of sensors during the gait session. The processor applies timestamps to frames of the video data of the user with respect to the measurement data based on the initialization signal to obtain time-synchronized gait visualization data and stores the time-synchronized gait visualization data within the application for subsequent retrieval and analysis.

In an embodiment, the mobile device is configured to playback the gait session comprising the time-synchronized gait visualization data through the user interface on the mobile device, wherein the user interface comprises the video data and a graphical chart indicating the gait of the user. The mobile device receives a user input to navigate to different timestamps within the gait session for analyzing video frames and associated timestamped measurement data. Further, the mobile device allows navigation to the different timestamps within the gait session based on the user input.

In an embodiment, the mobile device is configured to transmit the gait session comprising the time-synchronized gait visualization data to a remote server for retrieval and analysis at a later date or by a different user.

In an embodiment, the mobile device sends an initial timestamp to the assistive device via the short-range communication protocol while recording the video data of the user wearing the assistive device. The mobile device receives a response comprising a current time marking the start of the measurement data recording from the assistive device; the assistive device initializes the recording of the measurement data using the plurality of sensors at the time of sending the response. The mobile device determines a delta time representing a time difference between the initial timestamp and the current time marking the start of the measurement data recording. Further, the mobile device applies timestamps to frames of the video data of the user with respect to the measurement data based on the delta time. In an embodiment, the video data is trimmed to match the start time of recording of the measurement data by the plurality of sensors based on the delta time.

In an embodiment, the mobile device detects a unique sound generated and played by the assistive device at the moment of initiating the plurality of sensors for recording the measurement data; the unique sound is detected while recording the video data. Further, the mobile device applies timestamps to frames of the video data of the user by trimming the video data to align at least one frame of the video data corresponding to the detected unique sound.

In an embodiment, the mobile device sends a request to the remote server for gait analysis recommendations and receives an analysis of the user's gait data from the remote server. Based on this analysis, the mobile device then transmits personalized adjustments to the assistive device over the short-range communication protocol.

In an embodiment, the wireless connection between the mobile device and the assistive device is encrypted with a unique encryption key specific to the assistive device. In an embodiment, the mobile device receives a notification from the remote server to establish the gait session and receives an input from the user on the mobile device to initiate the gait session.

In an embodiment, the mobile device displays the user interface to configure an activity session to be performed by the user wearing the assistive device; the user interface comprises a plurality of activity parameters to be configured for the activity session to be performed by the user wearing the assistive device. Further, the mobile device performs the activity session to be performed by the user wearing the assistive device based on an input received from the user corresponding to the plurality of activity parameters. In an embodiment, the plurality of activity parameters comprises at least one of a session name, a session type, and a feedback survey. In an embodiment, the user interface displays a plurality of user details corresponding to the gait sessions of the user. The plurality of user details comprises at least one of a battery status of the assistive device, data synchronization status of the gait sessions, current mode indicating the physical status of the user and current status of the assistive device, and number of steps taken by the user for a period of time.

Embodiments disclosed herein provide a method for monitoring and visualizing a gait of a user. The method includes displaying by a mobile device a user interface via an application stored in the mobile device, wherein the user interface allows the user to connect to an assistive device worn by the user. The mobile device establishes a wireless connection with the assistive device via a short-range communication protocol, the assistive device including a plurality of sensors produces measurement data indicating movements of the user. The mobile device is configured to start a gait session to receive the measurement data from the plurality of sensors upon successful establishment of the wireless connection link; the gait session initializes the measurement data reception from the plurality of sensors. The mobile device of the mobile device receives an initialization signal from the assistive device confirming readiness of the plurality of sensors to transmit the measurement data. Further, the mobile device activates the camera to record video data of the user wearing the assistive device concurrently after receiving the initialization signal; the video data is recorded while receiving the measurement data from the plurality of sensors during the gait session. The mobile device applies timestamps to frames of the video data of the user with respect to the measurement data based on the initialization signal to obtain time-synchronized gait visualization data and stores the time-synchronized gait visualization data within the application for subsequent retrieval and analysis.

These and other aspects of the embodiments herein will be better appreciated and understood when considered in conjunction with the following description and the accompanying drawings. It should be understood, however, that the following descriptions, while indicating preferred embodiments and numerous specific details thereof, are given by way of illustration and not of limitation. Many changes and modifications may be made within the scope of the embodiments herein without departing from the scope thereof, and the embodiments herein include all such modifications.

### BRIEF DESCRIPTION OF FIGURES

These and other features, aspects, and advantages of the present disclosure are illustrated in the accompanying drawings, throughout which like reference letters indicate corresponding parts in the various figures. The embodiments herein will be better understood from the following description with reference to the drawings, in which:
FIG 1 illustrates a high-level overview of a system for monitoring and visualizing a user's gait according to the embodiments as disclosed herein.
FIG 2 is a block diagram of a mobile device for monitoring and visualizing the gait of the user according to embodiments as disclosed herein.
FIG 3 is a schematic diagram illustrating a method for monitoring and visualizing the gait of the user according to the embodiment as disclosed herein.
FIG 4A is illustrating a user interface of the mobile device depicting details of multiple users with multiple user IDs according to the embodiments as disclosed herein.
FIG 4B is illustrating the user interface of the mobile device scheduling a session for extracting time-synchronized gait visualization data of the user according to the embodiments as disclosed herein.
FIG 4C illustrates a scenario of connecting the mobile device with the user interface for performing an activity session according to the embodiments as disclosed herein.
FIG 4D is illustrating the user interface depicting details of the user according to the embodiments as disclosed herein.
FIG 4F is illustrating the user interface for recording the video data for the gait sessions according to the embodiments as disclosed herein.
FIG 4G is illustrating the user interface showing listed gait sessions for the user according to the embodiments as disclosed herein.
FIG 4H is illustrating a scenario of the user interface presenting the gait sessions to the user along with the timestamps to frames of the video data according to the embodiments as disclosed herein.
FIG 5 illustrates a use case of monitoring and visualizing the gait of the user according to the embodiments as disclosed herein, and
FIG 6 is a flow diagram illustrating the method of monitoring and visualizing the gait of the user according to the embodiments as disclosed herein.

It may be noted that to the extent possible, like reference numerals have been used to represent like elements in the drawing. Further, those of ordinary skill in the art will appreciate that elements in the drawing are illustrated for simplicity and may not have been necessarily drawn to scale. For example, the dimension of some of the elements in the drawing may be exaggerated relative to other elements to help to improve the understanding of aspects of the invention. Furthermore, the elements may have been represented in the drawing by conventional symbols, and the drawings may show only those specific details that are pertinent to the understanding the embodiments of the invention so as not to obscure the drawing with details that will be readily apparent to those of ordinary skill in the art having benefit of the description herein.

### DETAILED DESCRIPTION OF INVENTION

The embodiments herein and the various features and advantageous details thereof are explained more fully with reference to the non-limiting embodiments that are illustrated in the accompanying drawings and detailed in the following description. Descriptions of well-known components and processing techniques are omitted so as to not unnecessarily obscure the embodiments herein. Also, the various embodiments described herein are not necessarily mutually exclusive, as some embodiments can be combined with one or more other embodiments to form new embodiments. The term "or" as used herein, refers to a non-exclusive or, unless otherwise indicated. The examples used herein are intended merely to facilitate an understanding of ways in which the embodiments herein can be practiced and to further enable those skilled in the art to practice the embodiments herein. Accordingly, the examples should not be construed as limiting the scope of the embodiments herein.

As is traditional in the field, embodiments are described and illustrated in terms of blocks that carry out a described function or functions. These blocks, which are referred to herein as managers, units, modules, hardware components or the like, are physically implemented by analog and/or digital circuits such as logic gates, integrated circuits, microprocessors, microcontrollers, memory circuits, passive electronic components, active electronic components, optical components, hardwired circuits and the like, and optionally be driven by firmware and software. The circuits, for example, be embodied in one or more semiconductor chips, or on substrate supports such as printed circuit boards and the like. The circuits constituting a block be implemented by dedicated hardware, or by a processor (e.g., one or more programmed microprocessors and associated circuitry), or by a combination of dedicated hardware to perform some functions of the block and a processor to perform other functions of the block. Each block of the embodiments be physically separated into two or more interacting and discrete blocks without departing from the scope of the proposed method. Likewise, the blocks of the embodiments be physically combined into more complex blocks without departing from the scope of the proposed method.

The accompanying drawings are used to help easily understand various technical features and it is understood that the embodiments presented herein are not limited by the accompanying drawings. As such, the proposed method is construed to extend to any alterations, equivalents and substitutes in addition to those which are particularly set out in the accompanying drawings. Although the terms first, second, etc. used herein to describe various elements, these elements are not be limited by these terms. These terms are generally used to distinguish one element from another.

Embodiments disclosed herein provide a mobile device for monitoring and visualizing a gait of a user. The mobile device includes a camera, an I/O interface, a memory comprising an application and a processor connected to the memory. The processor is configured to display a user interface via the application, the user interface allows the user to connect to an assistive device worn by the user. The processor establishes a wireless connection with the assistive device via a short-range communication protocol, the assistive device including a plurality of sensors produces a measurement data indicating movements of the user. The processor is configured to start a gait session to receive the measurement data from the plurality of sensors upon successful establishment of the wireless connection link, the gait session initializes the measurement data reception from the plurality of sensors. The processor of the mobile device receives an initialization signal from the assistive device confirming readiness of the plurality of sensors to transmit the measurement data. Further the processor activates the camera to record a video data of the user wearing the assistive device concurrently after receiving the initialization signal, the video data is recorded while receiving the measurement data from the plurality of sensors during the gait session. The processor applies timestamps to frames of the video data of the user with respect to the measurement data based on the initialization signal to obtain time-synchronized gait visualization data, and stores the time-synchronized gait visualization data within the application for subsequent retrieval and analysis.

The embodiments disclosed herein revolutionize the approach to gait analysis by leveraging modern technology to provide a more comprehensive and user-friendly solution. The integration of a mobile device with an assistive wearable device, equipped with a multitude of sensors, facilitates a seamless and efficient method for monitoring and visualizing a user's gait. This innovative method begins with the user interface of a mobile application, which allows users to effortlessly connect their mobile devices to their assistive devices via short-range communication protocols. This connection is pivotal as it enables the mobile device to receive real-time measurement data from the sensors embedded in the assistive device, which capture intricate details of the user's movements.

Once the wireless connection link is successfully established, the mobile device initiates a gait session, marking the beginning of a synchronized data collection process. The assistive device sends an initialization signal to the mobile device, confirming the readiness of its sensors to transmit data. This signal not only triggers the reception of measurement data but also activates the mobile device's camera to record video footage of the user. The concurrent recording of video data alongside sensor data collection ensures that each frame of video is time-stamped and synchronized with the corresponding sensor data. This synchronization is crucial as it results in time-synchronized gait visualization data, which is stored within the application for future retrieval and analysis. The ability to correlate visual data with sensor measurements provides a richer, more nuanced understanding of the user's gait, enabling more precise assessments and interventions.

The proposed system offers a significant advancement over conventional gait analysis methods, which often suffer from limitations in data acquisition and processing. Traditional methods typically rely on a limited array of sensors, such as force plates or Inertial Measurement Units (IMUs), which can result in an incomplete representation of complex gait patterns. Additionally, these methods frequently struggle with issues of data synchronization and integration, leading to inconsistencies and potential inaccuracies in the analysis. The manual nature of data processing in conventional methods further compounds these challenges, introducing the potential for human error and variability in the interpretation of results. In contrast, the system described herein automates key aspects of the analysis process, reducing the time and effort required for data processing and minimizing the risk of errors. By providing a more comprehensive and efficient solution, this system holds the potential to transform the field of gait analysis, offering valuable insights that can enhance diagnosis, treatment, and rehabilitation efforts.

Moreover, the system's ability to facilitate remote monitoring and data access significantly enhances its utility and accessibility. Healthcare providers can initiate gait analysis sessions remotely and access the collected data from a centralized database, allowing for continuous monitoring of patient progress without the need for frequent in-person visits. This remote capability is particularly beneficial in the context of telemedicine, where timely interventions can be made based on real-time data insights. Furthermore, the system's capacity to identify specific gait events and generate notifications empowers users and healthcare providers to take proactive measures in addressing potential gait-related issues. By offering a comprehensive, automated, and accessible solution, the proposed system not only improves the accuracy and efficiency of gait analysis but also enhances the overall quality of care provided to users.

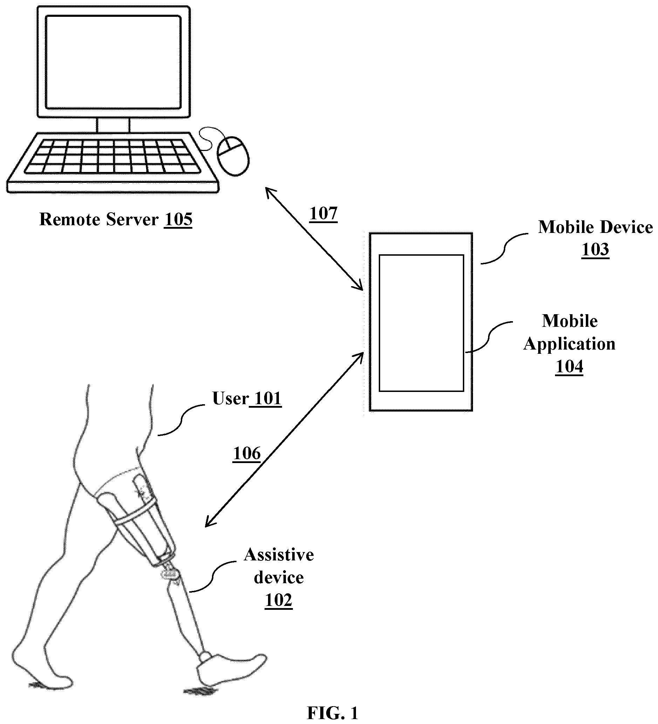

Referring now to the drawings, and more particularly to FIGS. 1 through 6 where similar reference characters denote corresponding features consistently throughout the figures, there are shown preferred embodiments.

FIG 1 illustrates a high-level overview of a system (100) for monitoring and visualizing the gait of a user (101) according to the embodiments as disclosed herein. The system (100) is a comprehensive platform for gait monitoring and analysis. The system (100) integrates wearable technology, mobile computing, cloud infrastructure, and advanced data processing techniques to provide a holistic view of human movement. The system (100) includes an assistive device (102) worn by the user (101), a mobile application (104) of a mobile device (103) for data capture and initial analysis, and a remote server (105) to control gait sessions of the user (101). The system (100) includes a cloud-based platform which is an online device portal for data storage, processing, and visualization. The cloud-based platform employs secure encryption protocols to ensure data privacy and integrity during transmission and storage. A different user or a Health Care Professional (HCP) of the online device portal and/or the remote server (103) with appropriate access can schedule a request to record an activity session of the gait of the user (101) by sending a notification to the patient's mobile device (103). The system (100) is designed to be scalable, allowing for the integration of additional sensors or devices as needed to accommodate various user requirements or advancements in sensor technology.

In an embodiment, the assistive device (102) equipped with the plurality of sensors collects real-time measurement data on the user (101) movements or the gait of the user (101). These sensors are strategically placed on the assistive device (102) to capture comprehensive data on the user's biomechanics, including limb acceleration, angular velocity, and orientation. Simultaneously, the mobile device (103) captures the video data of the user's gait. The video data is recorded at high frame rates to ensure detailed motion capture, which is crucial for analyzing rapid movements or subtle gait deviations. A critical component of the system (100) is the precise synchronization of the measurement data and the video data, ensuring accurate correlation between movement patterns and visual observations. This synchronization is achieved through a common time reference, such as a GPS-based timestamp or a network time protocol, enabling detailed analysis of gait parameters or gait metrics. The synchronized data allows for the creation of a comprehensive gait profile, which can be used for both diagnostic and therapeutic purposes.

In an embodiment, the assistive device (102) comprises a microprocessor coupled to the plurality of sensors. The microprocessor is responsible for preprocessing the sensor data, such as filtering noise and performing initial calculations, before transmission. The microprocessor facilitates transmission of the measurement data between the assistive device (102) and the mobile device (103) via the short-range communication protocols, including but not limited to Bluetooth Low Energy (BLE), Near Field Communication (NFE), Zigbee, Ultra-wideband (UWB), Infrared (IR), and Wi-Fi. These protocols are selected based on their power efficiency, data rate, and range, ensuring reliable communication in various environments. In an embodiment, the plurality of sensors include but are not limited to IMUs comprising accelerometers, gyroscopes, and magnetometers, wearable sensors such as pressure, electromyography sensors, and force sensors such as load cells, in-shoe pressure sensors, or force plates to measure ground reaction forces and pressure distribution, external sensors like cameras, optoelectronic sensors, and force plates, and additional sensor types including ultrasonic, radar, and GPS. Further, the plurality of sensors includes motion capture systems, both marker-based and markerless, are used to capture joint kinematics, while goniometers or inclinometers measure joint angles. Magnetometers assist in orientation sensing, and pressure and shear sensors analyze interface pressures between the prosthetic or orthotic device and the residual limb. Additionally, environmental sensors may detect external factors like terrain or slope, while wearable sensors embedded in smart textiles can monitor distributed forces, pressures, or limb health. Sensor fusion mechanisms integrate data from these sources for detailed and accurate gait analysis. These mechanisms employ machine learning techniques to adaptively improve the accuracy of gait assessments over time.

In an embodiment, the mobile device (103) initiates a gait session by pointing a camera of the mobile device (103) towards the user (101) wearing the assistive device (102) and records video data of the user (101) wearing the assistive device (102) while simultaneously receiving the measurement data indicating movements of the user (101) from the plurality of sensors of the assistive device (102). The mobile device (103) is equipped with advanced image processing capabilities to enhance video quality and extract relevant features in real-time. The mobile device (103) transmits both the measurement data and video data to the remote server (105) for further processing and analysis. The transmission is optimized to minimize latency and ensure that data is available for immediate analysis, which is crucial for applications requiring real-time feedback or intervention.

In an embodiment, the assistive device (102) may include but is not limited to an orthotic device, a prosthetic device, a smart device, or any other mobility-assistive device designed to support or enhance the user's physical functionality. The assistive device (102) can be equipped with adaptive control systems that adjust the level of assistance based on real-time feedback from the sensors. The assistive device (102) can be customized or configured to suit the specific needs of the user (102), such as providing stability, aiding in movement, or compensating for a physical limitation. Customization options may include adjustable components, modular designs, and software configurations that tailor the device's functionality to the user's unique gait characteristics and rehabilitation goals. In an embodiment, the assistive device (102) may be configured with or without microprocessors, wherein the plurality of sensors is operatively associated with the assistive device (102).

In an embodiment, some of the most advanced prosthetic devices include but not limited to a Ottobock C-Leg, a microprocessor-controlled knee prosthesis; the Ossur Proprio Foot, which adapts to uneven surfaces; the i-Limb Quantum, a myoelectric hand controlled by muscle signals; the Genium X3, a waterproof leg prosthesis with multi-terrain adaptability; and the Empower Ankle, a powered prosthetic ankle that mimics natural pushoff dynamics. In the field of orthotics, notable devices include the WalkAide System, which uses functional electrical stimulation for drop foot correction; the C-Brace, a microprocessor-controlled stance and swing orthosis; the HAL (Hybrid Assistive Limb), a robotic exoskeleton for rehabilitation; the Myomo MyoPro, a myoelectric orthosis that assists weak arm movements; and the Bioness L300 Go, which improves gait through adaptive foot-drop correction. These devices demonstrate significant advancements in mobility support and rehabilitation technologies.

In an embodiment, the collected data, including sensor readings and video data, is transmitted to a cloud-based platform or the remote server (105) for advanced processing and analysis. The cloud-based platform utilizes distributed computing resources to handle large volumes of data efficiently, enabling complex computations and data-intensive tasks. Sophisticated mechanisms are utilized to extract critical features or gait metrics from the measurement data, such as joint angles, forces, moments, and other biomechanical metrics. These mechanisms may incorporate machine learning models trained on extensive datasets to improve the accuracy and reliability of gait assessments. These features are integrated with video analysis to deliver a comprehensive understanding of the user's gait patterns. The system (100) is configured to detect key gait events, compute various gait parameters or gait metrics, and generate detailed visual representations, enabling accurate analysis and intuitive interpretation for clinicians, therapists, or users. The visual representations can be customized to highlight specific aspects of gait, such as symmetry, stride length, or cadence, providing valuable insights for clinical decision-making.

In an embodiment, the measurement data includes but not limited to positional data including joint angles (e.g., knee and ankle), limb segment positions (e.g., thigh and shank), and 3D spatial coordinates to track the trajectory of the foot during movement. Kinematic data such as thigh and shank angles, step and stride lengths, as well as swing and stance phase durations, provide insights into motion dynamics. Complementing this, kinetic data captures ground reaction forces (GRFs), joint torques (e.g., knee and ankle), and force distributions across specific foot regions like the heel, midfoot, and toes. Additionally, pressure data records plantar pressure distribution under the foot and localized pressure at skin-device interfaces. Temporal aspects of gait, including timing of gait events (heel strike and toe-off), cadence (steps per minute), and gait cycle duration, are also measured. Inertial data from sensors like accelerometers, gyroscopes, and magnetometers offer linear acceleration, angular velocity, and orientation angles for precise motion tracking. Muscle engagement is assessed via electromyographic (EMG) signals from muscles such as the quadriceps and gastrocnemius. Interaction with the environment is monitored using data on foot clearance during the swing phase and surface type detection. The energy and effort exerted during movement are estimated through metrics such as metabolic energy and power output at joints. Finally, feedback data, including skin temperature at the device interface and device-induced vibrations or haptic signals, provides additional context. This comprehensive sensor data forms the foundation for detailed gait visualization, enabling precise adjustments to prosthetic or orthotic devices for optimal performance.

In an embodiment, the cloud-based platform or the remote server (105) serves as a central repository for storing and managing collected data. This centralized approach enables secure data backup, efficient data sharing, and collaboration among healthcare providers. The platform employs robust access control mechanisms to ensure that only authorized users can access sensitive data. Additionally, the platform facilitates large-scale data analysis and the development of predictive models or generative Artificial Intelligence (AI) models to identify potential gait abnormalities or treatment outcomes. These models can be used to simulate various intervention scenarios, providing clinicians with evidence-based recommendations for optimizing patient care and improving rehabilitation outcomes.

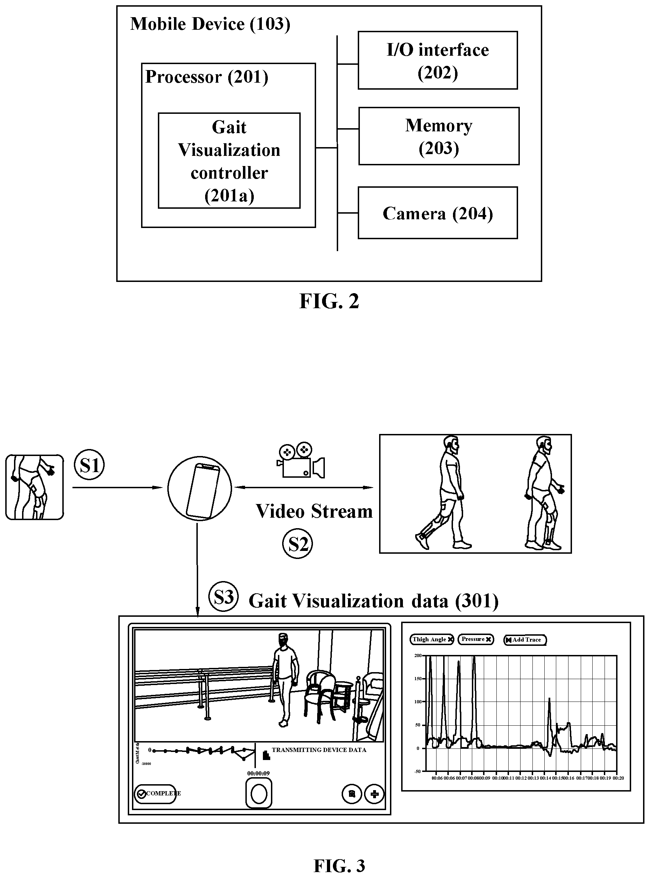

FIG. 2 is a block diagram of the mobile device (103) for monitoring and visualizing the gait of the user (101), according to embodiments as disclosed herein. With reference to FIG 2, the mobile device (103) can encompass a diverse range of devices, including but not limited to laptops, palmtops, desktops, mobile phones, smart phones, Personal Digital Assistants (PDAs), tablets, wearable devices, smart watches, Internet of Things (IoT) devices, virtual reality devices, foldable devices, flexible devices, display devices, and immersive systems. In an embodiment, the mobile device (103) includes a memory (203), a processor (201), an Input/ Output (I/O) interface (202), and a camera (204). The processor (201) includes a gait visualization controller (201a).

The memory (203) is configured to store instructions to be executed by the processor (203), and the mobile application (104). The memory (203) can include non-volatile storage elements. Examples of such non-volatile storage elements may include magnetic hard discs, optical discs, floppy discs, flash memories, or forms of electrically programmable memories (EPROM) or electrically erasable and programmable (EEPROM) memories. In addition, the memory (203) may, in some examples, be considered a non-transitory storage medium. The term "non-transitory" may indicate that the storage medium is not embodied in a carrier wave or a propagated signal. However, the term "non-transitory" should not be interpreted that the memory (203) is non-movable. In some examples, the memory (203) is configured to store larger amounts of information. In certain examples, a non-transitory storage medium may store data that can, over time, change (e.g., in Random Access Memory (RAM) or cache).

The processor (201) may include one or a plurality of processors. The one or the plurality of processors may be a general-purpose processor, such as a central processing unit (CPU), an application processor (AP), or the like, a graphics-only processing unit such as a graphics processing unit (GPU), a visual processing unit (VPU), and/or an AI-dedicated processor such as a neural processing unit (NPU). The processor (203) may include multiple cores and is configured to execute the instructions stored in the memory (203).

The I/O interface (202) transmits the information between the memory (203) and external peripheral devices. The peripheral devices are the input-output devices associated with the network apparatus. The I/O interface (202) receives several information from plurality of UEs, network devices, server and the like.

In an embodiment, the gait visualization controller (201a) of the processor (206) communicates with the I/O interface (202) and memory (203) for monitoring and visualizing the gait of the user (101). The gait visualization controller (201a) is configured to display a user interface via the application (104). The user interface allows the user (101) to connect to the assistive device (102) worn by the user (101). The gait visualization controller (201a) establishes a wireless connection with the assistive device (102) via the short-range communication protocol, such as Bluetooth Low Energy (BLE) or Zigbee, ensuring minimal power consumption and efficient data transfer. The assistive device (102), including the plurality of sensors such as accelerometers, gyroscopes, and magnetometers, produces the measurement data indicating movements of the user (101). These sensors are strategically placed on the assistive device (102) to capture comprehensive motion data, including stride length, cadence, and joint angles. The gait visualization controller (201a) is configured to start the gait session to receive the measurement data from the plurality of sensors upon successful establishment of the wireless connection link. The gait session initializes the measurement data reception from the plurality of sensors, ensuring that all data streams are synchronized from the onset. The gait visualization controller (201a) of the mobile device (103) receives an initialization signal from the assistive device (102) confirming readiness of the plurality of sensors to transmit the measurement data. Further, the gait visualization controller (201a) activates the camera (204) to record a video data of the user (101) wearing the assistive device (102) concurrently after receiving the initialization signal. The video data is recorded at a high frame rate to capture detailed motion analysis while receiving the measurement data from the plurality of sensors during the gait session. The gait visualization controller (201a) applies timestamps to frames of the video data of the user (101) with respect to the measurement data based on the initialization signal to obtain time-synchronized gait visualization data and stores the time-synchronized gait visualization data within the application (104) for subsequent retrieval and analysis. This synchronization allows for precise correlation between visual and sensor data, facilitating advanced gait analysis.

In an embodiment, the gait visualization controller (201a) is configured to receive a user input to navigate to different timestamps within the gait session for analyzing video frames and associated timestamped measurement data. The user interface provides intuitive controls, such as a timeline slider and playback buttons, enabling the user to easily access specific moments of interest within the gait session. Further, the gait visualization controller (201a) is configured to playback the gait session comprising the time-synchronized gait visualization data (401) through the user interface on the mobile device (103). The user interface comprises the video data and a graphical chart indicating the gait of the user (101), such as step symmetry, speed, and balance metrics. The graphical chart is dynamically updated to reflect real-time changes as the user navigates through the session. The gait visualization controller (201a) allows navigation to the different timestamps within the gait session based on the user input, providing options for slow-motion playback and frame-by-frame analysis to enhance the user's understanding of their gait patterns.

In an embodiment, the gait visualization controller (201a) uploads the gait session comprising the time-synchronized gait visualization data to a remote server (105) for retrieval and analysis at a later date or by a different user (101). The data is encrypted during transmission to ensure privacy and security, adhering to industry standards such as AES-256 encryption. The remote server (105) is equipped with advanced data processing capabilities, allowing for in-depth analysis using machine learning mechanisms to identify potential gait abnormalities or improvements over time. Users can access their data through a secure web portal, where they can view detailed reports and share insights with healthcare professionals or caregivers. This remote access feature facilitates collaborative analysis and supports telemedicine applications, enabling users to receive expert feedback without the need for in-person consultations.

In an embodiment, the gait visualization controller (201a) sends a firmware update request message of the assistive device (102) to the remote server (105) and receives a firmware update response message from the remote server (105). The firmware update response message includes a firmware to be updated at the assistive device (102). The gait visualization controller (201a) transmits the firmware to the assistive device (102) over the short-range communication protocol, ensuring a secure and reliable update process. The microprocessors in the assistive device (102) have the ability to be updated over-the-air via the mobile device (103), utilizing a dual-bank memory architecture to prevent data loss during the update. A user with relevant permissions in the mobile device (103) can request the latest device firmware from the remote server (105), and the firmware is transferred over the secure communication to the assistive device (102). Once transferred, the new firmware is flashed to the assistive device (102) using a bootloader mechanism that verifies the integrity of the firmware before installation. The assistive device (102) is updated by a trained professional and doesn't happen automatically, ensuring that any potential issues are addressed promptly and that the device remains in optimal working condition.

In an embodiment, the gait visualization controller (201a) displays the user interface to configure an activity session to be performed by the user (101) wearing the assistive device (102). The user interface comprises a plurality of activity parameters to be configured for the activity session to be performed by the user (101) wearing the assistive device (102). These parameters include the type of activity, duration, intensity level, and specific goals such as distance or step count. Further, the gait visualization controller (201a) performs the activity session to be performed by the user (101) wearing the assistive device (102) based on an input received from the user corresponding to the plurality of activity parameters. The controller provides real-time feedback and guidance through audio cues or haptic feedback, helping the user maintain proper form and achieve their activity goals. Additionally, the system can adapt the activity session dynamically based on the user's performance, offering personalized recommendations to enhance the effectiveness of the training session.

The gait visualization controller (201a) is an inventive hardware component that is incorporated into the mobile device (103) through processing circuitry, comprising of logic gates, integrated circuits, microprocessors, microcontrollers, memory circuits, passive and active electronic components, optical components, hardwired circuits, or similar technologies. These circuits can be manifested in one or more semiconductor chips or on substrate supports such as printed circuit boards.

At least one of the plurality of components of the gait visualization controller (201a) may be implemented through an AI model. A function associated with the AI model may be performed through the memory (203) and the gait visualization controller (201a). The one or a plurality of processors controls the processing of the input data in accordance with a predefined operating rule or the AI model stored in the non-volatile memory and the volatile memory. The predefined operating rule or artificial intelligence model is provided through training or learning.

Here, being provided through learning means that, by applying a learning process to a plurality of learning data, a predefined operating rule or AI model of a desired characteristic is made. The learning may be performed in a device itself in which AI according to an embodiment is performed, and/or may be implemented through a separate server/system.

The AI model may consist of a plurality of neural network layers. Each layer has a plurality of weight values and performs a layer operation through calculation of a previous layer and an operation of a plurality of weights. Examples of neural networks include, but are not limited to, convolutional neural network (CNN), deep neural network (DNN), recurrent neural network (RNN), restricted Boltzmann Machine (RBM), deep belief network (DBN), bidirectional recurrent deep neural network (BRDNN), generative adversarial networks (GAN), and deep Q-networks.

The learning process is a method for training a predetermined target device (for example, a robot) using a plurality of learning data to cause, allow, or control the target device to make a determination or prediction. Examples of learning processes include, but are not limited to, supervised learning, unsupervised learning, semi-supervised learning, or reinforcement learning.

Whilst FIG 2 depicts the hardware components of the mobile device (103), it should be noted that alternative embodiments are not confined to these elements. The mobile device (103) may comprise a greater or lesser number of hardware components in other embodiments. Additionally, the labels or names assigned to these elements are purely for illustrative purposes and do not restrict the scope of the invention. Furthermore, it is possible for one or more components to be merged together to perform the same or a substantially similar function.

In the embodiment disclosed, the method for monitoring and visualizing the gait of the user (101) leverages the integration of mobile technology with assistive devices to enhance both user experience and healthcare provider (HCP) insights. At step S1, the mobile device (103) establishes a wireless connection with the assistive device (102) via a short-range communication protocol, such as Bluetooth or NFC. This connection facilitates seamless data exchange, ensuring that the mobile device (103) can receive real-time sensor data from the assistive device (102). The assistive device (102) is equipped with a plurality of sensors that capture various movement parameters of the user (101), such as acceleration, angular velocity, and spatial orientation. This sensor data is crucial for accurately analyzing the user's gait and identifying any irregularities or areas for improvement.

At step S2, the mobile device (103) initiates the gait session by utilizing its camera (204) to record video data of the user (101) while they are wearing the assistive device (102). This video recording is synchronized with the sensor data received from the assistive device (102), allowing for a comprehensive analysis of the user's gait. The synchronization is achieved by applying timestamps to the frames of the video data, aligning them with the corresponding measurement data from the sensors. This process ensures that each frame of the video is accurately matched with the precise movements of the user (101), providing a detailed visualization of their gait. The recorded video data, along with the sensor measurements, are stored within the application (104) on the mobile device (103) for further analysis and retrieval.

At step S3, the mobile device (103) analyzes the gait of the user (101) by calculating various gait parameters, such as stride length, cadence, and balance. These parameters are crucial for assessing the effectiveness of the assistive device (102) and identifying any potential issues in the user's gait. The mobile device (103) can provide live feedback through a gait visualizer, which displays the time-synchronized gait visualization data (301) and calculated gait parameters. This visualizer can be used by healthcare providers to optimize the fitting and alignment of the assistive device (102), ensuring that it provides maximum support and comfort to the user (101). Additionally, the visualizer offers the ability to record and playback the gait session, allowing for detailed analysis and comparison over time. This feature provides valuable insights and metrics for both the user (101) and the healthcare provider, facilitating ongoing monitoring and adjustment of the assistive device (102) to enhance the user's mobility and quality of life.

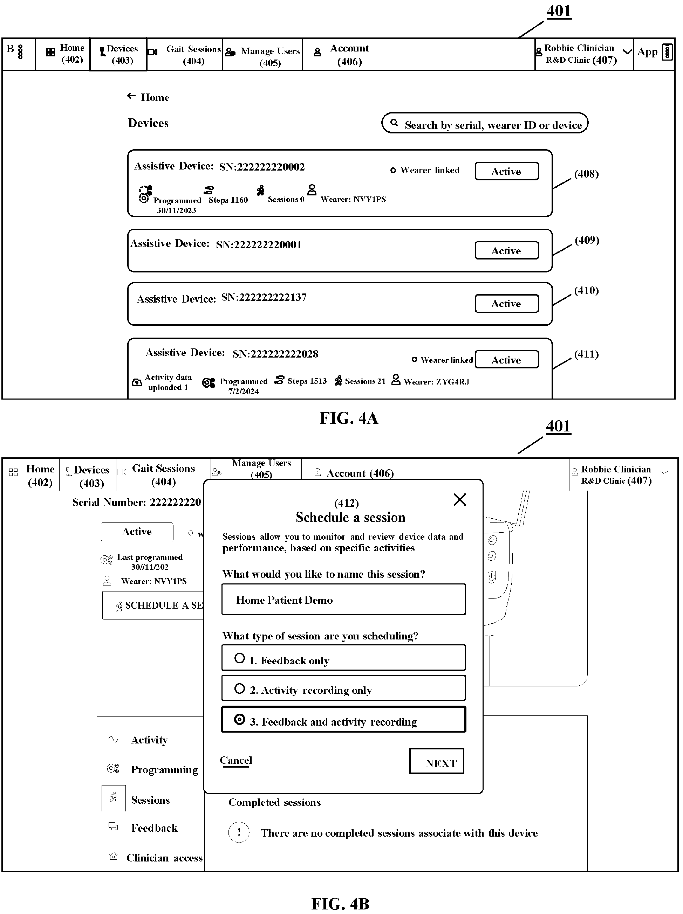

FIG 4A is illustrating a user interface (401) of the mobile device (103) depicting details of multiple users with multiple user IDs according to the embodiments as disclosed herein. The user interface (401) includes a list of users (408-411) with unique IDs for easy identification. The user interface (401) includes navigation options such as Home (402), which serves as a dashboard or main landing page summarizing key information or actions; Devices (403) with the list of users (408-411) with unique IDs for managing connected devices; Gait Sessions (404), for accessing data related to the gait sessions and the gait analysis; Manage Users (405), for adding, editing, or deleting user profiles; and Account Information (406), for managing personal settings and preferences. Further the user interface (401) includes name and place of clinician (407).

Further, the user interface (401) provides a visual representation of movement analysis, device analysis, and expert analysis. The movement analysis section offers insights into mobility-related metrics, including fall detection, gait analysis, and movement tracking, ensuring real-time monitoring and safety. This section utilizes advanced mechanisms to process accelerometer and gyroscope data, providing detailed reports on stride length, cadence, and symmetry. The device analysis section focuses on assistive device parameters such as suspension, limb volume measurement, tension and pressure monitoring, and alignment checks, helping to optimize device performance and customization. Sensors embedded in the assistive device collect data on pressure distribution and alignment, which are crucial for preventing discomfort and injury. The expert analysis feature enables healthcare professionals to provide informed recommendations, including adjustments to medication or therapy based on the collected data and gait trends. This feature integrates machine learning models that predict potential health issues, allowing for proactive healthcare management. The users or healthcare professionals can utilize the configuration settings to set up new gait sessions, tailoring the system to specific activities or environments. The event detection section records adverse events or device malfunctions, storing sensor and video data to facilitate thorough investigation and timely intervention. Additionally, the gait sessions section allows users to list, sort, select, or delete recorded sessions, ensuring efficient data management for reviews or analysis. The user interface integrates all these features into a single cohesive platform, enabling seamless navigation, real-time updates, and easy access to historical data. This intuitive design supports the HCPs and users in monitoring, analyzing, and optimizing gait and device functionality while promoting personalized healthcare interventions.

FIG 4B is illustrating the user interface (401) of the mobile device (103) scheduling a session (412) for extracting the time-synchronized gait visualization data (301) of the user (101) according to the embodiments as disclosed herein. The process of scheduling the session (412) involves selecting different session types based on the specific gait data needed, such as walking or running patterns, and the level of detail required. Users can choose from predefined templates or customize session parameters to focus on specific metrics like speed, balance, or endurance. The session unfolds in multiple stages where various types of data are collected at different points. One stage may involve initial data gathering, while subsequent stages could include feedback loops where the system evaluates or refines the data. These stages are designed to capture comprehensive data sets, including environmental factors like surface type and incline, which can affect gait. Feedback sections help ensure the accuracy and quality of the gait data by allowing real-time adjustments or corrections based on the information being collected. In essence, scheduling a session (412) involves setting up a structured process for extracting precise and meaningful gait data through different session options, stages, and feedback mechanisms. This structured approach ensures that the data collected is both relevant and actionable, providing a solid foundation for subsequent analysis and intervention.

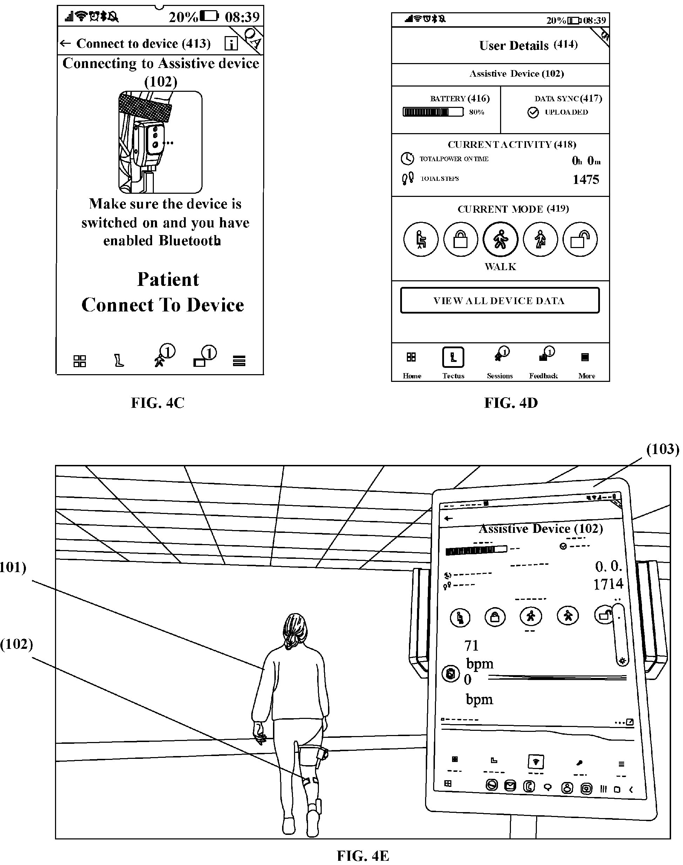

FIG 4C illustrates a scenario of connecting (413) the mobile device (103) with the user interface (401) for performing the activity session according to the embodiments as disclosed herein. The connection process involves establishing a secure link between the mobile device (103) and the assistive device (102) using Bluetooth or another short-range communication protocol. This connection ensures that the measurement data is transmitted in real-time, allowing for immediate feedback and adjustments during the activity session. The user interface (401) guides the user (101) through the connection process, providing visual cues and notifications to confirm successful pairing. Once connected, the system (100) continuously monitors the status of the connection, alerting the user to any disruptions or issues that may arise. This robust connectivity framework is essential for maintaining the integrity of the data collected during the session, ensuring that all relevant metrics are accurately captured and recorded.

FIG 4D is illustrating the user interface (401) depicting user details (414) according to the embodiments as disclosed herein. The user interface (401) displays personal details such as user ID, user name, etc., activity status (current mode), total steps, gait sessions, session details with data and time, configuration settings, and event detection. In an embodiment, the user interface (401) displays a plurality of user details corresponding to the gait sessions of the user (101). The plurality of user details includes at least one of a battery status (416) of the assistive device (102), data synchronization (417) status of the gait sessions, current mode (419) indicating the physical status of the user (101), and current activity (418) of the assistive device (102), and number of steps taken by the user (101) for a period of time. The interface also provides alerts for low battery or data synchronization issues, ensuring that users can address these promptly to avoid data loss. In an embodiment, the plurality of activity parameters comprises at least one of a session name, a session type, and a feedback survey. The feedback survey allows users to provide subjective input on their experience, which can be correlated with objective data to enhance the accuracy of the analysis. This comprehensive display of user information supports informed decision-making and personalized care planning.

FIG 4E is illustrating the user interface (401) for collecting the measurement data for the gait sessions according to the embodiments as disclosed herein. The interface (401) is designed to guide users through the data collection process, providing step-by-step instructions and visual indicators to ensure accurate measurements. It supports various data types, including kinematic, kinetic, and spatiotemporal parameters, which are critical for a thorough gait analysis. The system (100) employs advanced filtering techniques to eliminate noise and enhance the quality of the data collected. Users can view real-time graphs and charts that depict their gait metrics, allowing for immediate assessment and adjustments if necessary. This interactive approach to data collection not only improves user engagement but also enhances the reliability of the data gathered.

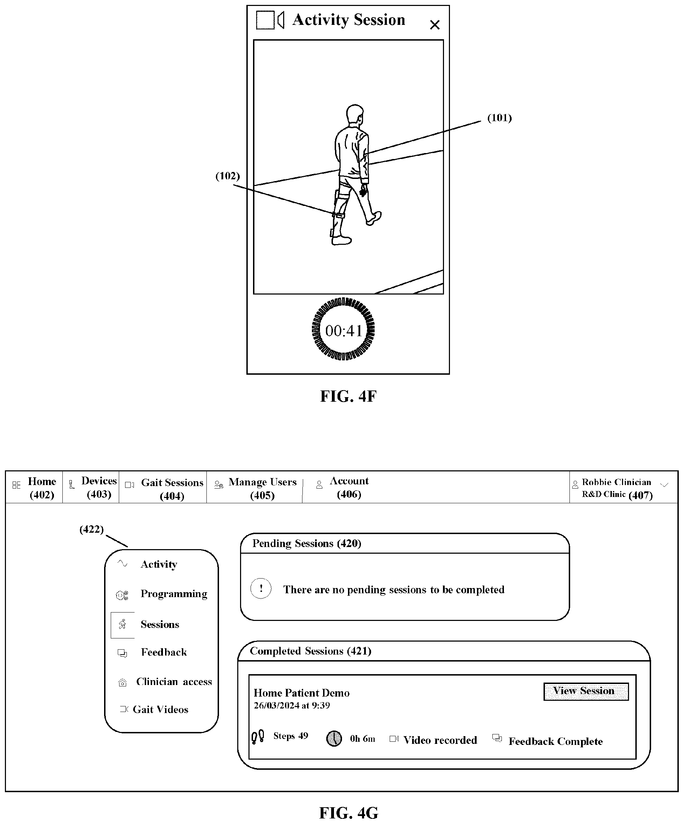

FIG 4F is illustrating the user interface (401) for recording the video data for the gait sessions according to the embodiments as disclosed herein. The video recording feature is integrated with the measurement data collection, providing a synchronized view of the user's gait. High-definition video capture ensures that even subtle movements are recorded with clarity, which is essential for detailed analysis. The interface (401) allows the users to adjust camera settings, such as resolution and frame rate, to suit different environments and lighting conditions. The users can also annotate video recordings with notes or markers to highlight specific events or observations. This capability enhances the utility of the video data, making it a valuable tool for both users and healthcare professionals in assessing and improving gait.

FIG 4G is illustrating the user interface (401) showing listed gait sessions for the user (101) according to the embodiments as disclosed herein. The user interface (401) provides a list of pending sessions (420) to be performed by the user (101), and the list of completed sessions (421). Further, the user interface (401) includes a list of navigation features (422) including activity, programming, sessions, feedback, clinician access, and the gait videos. In an embodiment, the system (100) enables the recording of the gait sessions in any environment-clinic, home, or outdoors-allowing for the collection of real-world data in natural settings. The system (100) supports remote appointments, such as physiotherapy or routine reviews, reducing the need for in-person visits, saving time, and lowering transportation costs. The interface provides options for filtering and sorting sessions based on various criteria, such as date, location, or session type, facilitating easy access to relevant data. In case of device malfunctions, the mobile application (104) helps assess the issue's severity and urgency. By integrating patient feedback with objective gait data, the system (100) provides valuable contextual insights, enhancing personalized care and informed decision-making. This integration of subjective and objective data allows for a more comprehensive understanding of the user's condition and progress.

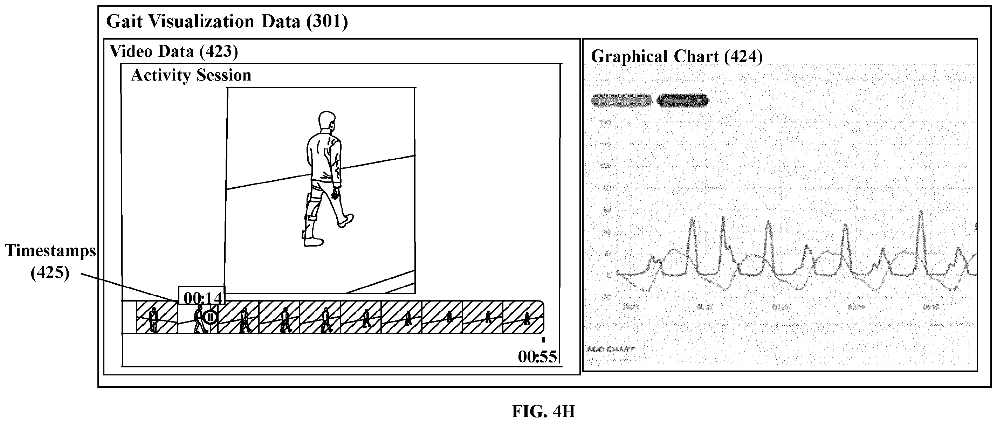

FIG 4H is illustrating a scenario of the user interface (401) presenting the gait sessions to the user (101) along with timestamps (425) to frames of video data (423) according to the embodiments as disclosed herein. In an embodiment, a time-synchronized gait visualization data (301) is obtained after applying timestamps (425) to frames of the video data of the user (101) with respect to the measurement data based on the initialization signal. The time-synchronized gait visualization data (301) further includes a graphical chart (424) illustrating the gait parameters. This synchronization ensures that each frame of video data (423) corresponds precisely to the measurement data, allowing for accurate analysis of gait patterns. In an embodiment, the mobile device (103) sends an initial timestamp to the assistive device (102) via the short-range communication protocol while recording the video data of the user (101) wearing the assistive device (102), and the mobile device (103) receives a response comprising a current time marking the start of the measurement data recording from the assistive device (102). The assistive device (102) initializes recording of the measurement data using the plurality of sensors at the time of sending the response. Further, the mobile device (103) determines a delta time representing a time difference between the initial timestamp and the current time marking the start of the measurement data recording. The mobile device (103) applies timestamps to frames of the video data of the user (101) with respect to the measurement data based on the delta time. In an embodiment, the video data is trimmed to match the start time of recording of the measurement data by the plurality of sensors based on the delta time. This precise alignment of video and measurement data is crucial for identifying and analyzing specific gait events.

In an embodiment, the mobile device (103) detects a unique sound generated and played by the assistive device (102) at the moment of initiating the plurality of sensors for recording the measurement data. The unique sound is detected while recording the video data. Further, the mobile device (103) applies timestamps to frames of the video data of the user (101) by trimming the video data to align at least one frame of the video data corresponding to the detected unique sound. This method provides an additional layer of synchronization, ensuring that the video data is perfectly aligned with the measurement data.

In an embodiment, the mobile device (103) identifies an event associated with the user (101) by analyzing the time-synchronized gait visualization data (301) and video data of the user (101). Upon determining the event, a notification is sent to the user (101) to inform them about the detected event. In an embodiment, the mobile device (103) determines behavioral data of the user (101) wearing the assistive device (102) based on the activity information, the time-synchronized gait visualization data (301), and the video data of the user (101), and determines the health condition of the user (101) in real-time by correlating the behavioral data with contextual information based on feedback of the user (101) wearing the assistive device (102). Additionally, the mobile device (103) is configured to generate alerts or notifications based on predefined criteria or the behavioral data, such as identifying abnormal gait patterns, detecting irregularities in movement, or assessing potential fall risks based on the gait visualization. These proactive notifications aim to enhance user safety and enable timely interventions. The system's ability to correlate behavioral data with contextual information provides a comprehensive view of the user's health, supporting more accurate and effective interventions.

In an embodiment, the mobile application (104) provides a user-friendly interface for initiating gait recordings, reviewing captured data, and accessing basic analysis results. Users (101) can easily start and stop recordings, adjust recording settings, and view real-time visualizations of their gait. The application includes interactive tutorials and help sections to assist users in navigating the interface and utilizing its features effectively. This accessibility ensures that users of all technical skill levels can benefit from the system's capabilities, promoting widespread adoption and use.

In an embodiment, the gait metrics or gait parameters encompass various measurable parameters that analyze walking patterns, providing quantitative insights into gait quality, abnormalities, and rehabilitation progress. Key temporal metrics include cadence (steps per minute), step time, stride time, stance time, swing time, and periods of double and single support. Spatial metrics cover step length, stride length, step width, and foot clearance. Kinematic metrics focus on joint angles, range of motion (ROM), and body segment trajectories, while kinetic metrics measure ground reaction forces (GRF), joint moments, and joint power during movement. Symmetry and balance metrics assess gait symmetry, center of mass (CoM) displacement, and stability. Performance metrics like walking speed, energy expenditure, and gait efficiency provide insights into mobility and effort. Event-based metrics identify critical phases such as heel strike, toe-off, and gait cycle divisions, while contextual metrics examine terrain adaptation and activity-specific variations. Together, these metrics enable the HCPs to evaluate impairments, track recovery, optimize assistive devices, and tailor interventions to enhance mobility and quality of life.

In an embodiment, the gait sessions, comprising the time-synchronized gait visualization data (301) along with corresponding video data recorded over a specified period, can be replayed on the mobile device (103), a web browser connected to the remote server (105) or cloud-based platform. Additionally, these gait sessions can be securely shared with authorized users, such as clinicians, therapists, or caregivers, for review and analysis at a later date. This functionality enhances collaboration and enables detailed assessments across multiple stakeholders, supporting improved decision-making and personalized care.

In an embodiment, a user or HCP with authorized access to the remote server (105) or an online portal can schedule a request, which triggers a notification to the user's mobile device (103). An activity may include a combination of device data, video recordings, and user (101) feedback questionnaires, including scientifically validated instruments. This functionality provides flexibility to the HCPs to gather not only behavioral data from the user (101) and the assistive device (102) but also contextual insights into the user's subjective experiences and feelings at the time of the session, enhancing the relevance of the collected data.

In an embodiment, the HCPs doesn't have to rely on memory of how the user (101) walked or moved. The gait sessions provide a visual record of the user (101) wearing the assistive device (102), evidencing the set-up, function and efficacy of the assistive device (102) in a variety of environments. The system (100) provides a snapshot of the assistive device (102), its performance and functionality at that moment in time, that can be compared against previous or subsequently collected data. This provides an accurate, objective means of documenting the effect of adjustments, prescription changes and therapy on mobility and device functionality over time.

In an embodiment, pause, rewind and replay of the synchronized video data and measurement data gives the HCP the ability to perform more in-depth analysis of how the assistive device (102) is performing for the patient and identify on a frame-by-frame basis whether it is optimally set up and functioning as intended. This data can be used during trouble-shooting, comparing data provided by onboard sensors with that collected by other means, ensuring rapid appraisal of potential discrepancies and device issues. The recorded gait sessions can be shared between the HCPs for peer review and analysis. Consent for sharing is provided by the user (101) per-recording to ensure data privacy regulations are complied with.

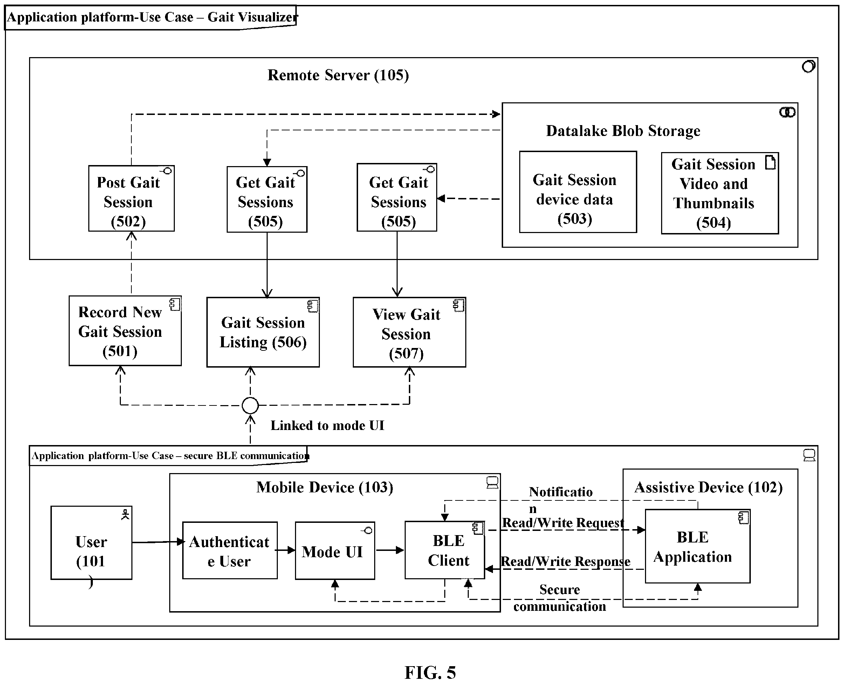

FIG. 5 illustrates a use case of monitoring and visualizing the gait of the user (101), according to the embodiments as disclosed herein. The user (101) is authenticated to access the mobile device (103) for performing the gait sessions. Through the user interface (401) the mobile device (103) connects to the assistive device (102) via the BLE. Record new gait session (501) block initiates the gait session which in turn initiated by pointing the camera (204) towards the user (101) wearing the assistive device (102) while simultaneously receiving the measurement data from the assistive device (102). Once the gait session is completed, the mobile device synchronizes the video data and the measurement data to obtain the time-synchronized gait visualization data (301) based on the initialization signal received from the assistive device (102). The time-synchronized gait visualization data (301) along with the video data and the measurement data is stored as the gait sessions. Theses gait sessions are transmitted to the remote server (105). Post gait session (502) block further analyses the gait sessions to generate various gait parameters on the gait sessions. The gait sessions and other data related to gait analysis of the user (101) are stored in a datalake blob storage as gait session and device data (503), and gait session video and thumbnails (504). Get gait session (505) block extracts the data stored on the datalake blob storage for further analysis by the user (101) or the HCP through Gait session listing (506), and the vies gait session (507).

In an embodiment, the mobile device (103) or other user who has access to the remote server (105) retrieves the video data and the time synchronized gait visualization from the remote server (105) and presents them through an intuitive user interface or a dedicated mobile application (104). The user interface (401) is designed to display the gait visualization alongside the video data in a synchronized manner, allowing user (101) to seamlessly analyze the relationship between real-time movements and gait metrics. Interactive features, such as playback controls, zooming, and annotation tools, can enhance the user experience, enabling users to highlight specific gait phases or share insights. This user-friendly interface ensures that clinicians, therapists, or users can efficiently review and interpret the gait data directly on the mobile device (103) or any other user device with access to the remote server (105).

In an embodiment, the mobile device (103) receives an input from the user (101) wearing the assistive device (102) on the mobile device (105) to capture the video data. The mobile device (103) automatically initiates the gait visualizer recording by pointing the camera (204) of the mobile device (103) towards the user (101) wearing the assistive device (102) to record the video data of the user (101) while simultaneously receiving the measurement data from the plurality of sensors based on the input from the user (101).

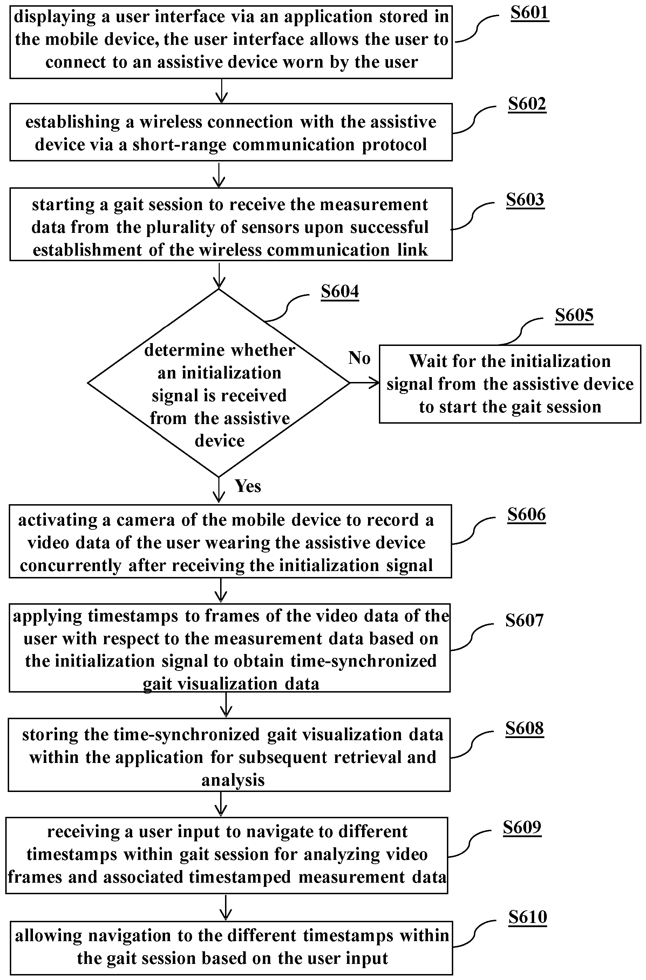

FIG 6 is a flow diagram illustrating the method of monitoring and visualizing the gait of the user (101) according to the embodiments as disclosed herein. At step S601, the mobile device (103) displays the user interface (401) via the application (104). The user interface (401) allows the user (101) to connect to the assistive device (102) worn by the user (101).

At step S602, the mobile device (103) establishes the wireless connection with the assistive device (102) via the short-range communication protocol. The assistive device (102), including the plurality of sensors, produces the measurement data indicating movements of the user (101).

At step S603, the mobile device (103) starts a gait session to receive the measurement data from the plurality of sensors upon successful establishment of the wireless connection link. The gait session initializes the measurement data reception from the plurality of sensors.

At step S604, the mobile device (103) determines whether the initialization signal is received from the assistive device (102) confirming readiness of the plurality of sensors to transmit the measurement data.

At step S605, the mobile device (103) waits for the initialization signal from the assistive device (102) to start the gait session when the initialization signal is not received from the assistive device (102) confirming readiness of the plurality of sensors to transmit the measurement data.

At step S606, when the mobile device (103) receives the initialization signal from the assistive device (102) confirming readiness of the plurality of sensors to transmit the measurement data, the mobile device (103) activates the camera (204) to record the video data of the user (101) wearing the assistive device (102) concurrently after receiving the initialization signal. The video data is recorded while receiving the measurement data from the plurality of sensors during the gait session. In an embodiment, the mobile device (103) is fixed at a distance or can be handled by another user or a caretaker to capture the video data of the user wearing the assistive device (102).

At step S607, the mobile device (103) applies timestamps to frames of the video data of the user (101) with respect to the measurement data based on the initialization signal to obtain time-synchronized gait visualization data (301).

At step S608, the mobile device (103) stores the time-synchronized gait visualization data (301) within the application (104) for subsequent retrieval and analysis. Further, the mobile device (103) transmits the time-synchronized gait visualization data (301) to the remote server (105) for retrieval and analysis at a later date or by a different user or the HCPs. In an embodiment, the remote server (105) or the online device portal provides the HCPs with comprehensive tools to oversee various aspects of user management and gait visualization data (301). The online portal displays a list of multiple user IDs, allowing the HCPs or authorized personnel to easily access detailed information associated with each user. This includes gait visualization data (301), which combines synchronized measurement data and video recordings to provide an in-depth analysis of user mobility patterns and device interactions. In addition to the gait data, the portal showcases the activity status of users, providing real-time updates or summaries of their current movement or rehabilitation activities. It also includes account details of the HCP, ensuring transparency and enabling personalized recommendations based on the user's progress. The portal is equipped with features for managing users, including options to add, edit, or remove users from the system. This allows HCPs to efficiently handle multiple user profiles and customize support or recommendations as needed. Overall, the remote server (105) or online device portal acts as a powerful hub for the HCPs to monitor, analyze, and manage user data, enabling data-driven insights and improved decision-making in the rehabilitation process.

At step S609, the mobile device (103) receives a user input to navigate to different timestamps within the gait session for analyzing video frames and associated timestamped measurement data. The mobile device (103) is configured to playback the gait session comprising the time-synchronized gait visualization data (301) through the user interface (401) on the mobile device (103). The user interface (401) comprises the video data and the graphical chart indicating the gait of the user (101).

At step S610, the mobile device (103) allows navigation to the different timestamps within the gait session based on the user input. In an embodiment, the system (100) provides additional data points by analyzing how the assistive device (102) operates in real-world conditions as the user (101) moves, helping the HCPs assess whether the assistive device (102) is properly configured. This enables the HCPs to fine-tune the assistive device (102) or guide the user (101) on optimizing their movements to improve gait. Furthermore, the system (100) benchmarks the user (101) against similar users with comparable demographics, amputation levels, and co-morbidities, offering insights into asymmetries and rehabilitation progress. The system (100) may also recommend physiotherapy exercises, hardware adjustments, or alternative devices proven effective for similar patients, enhancing personalized rehabilitation strategies.

New software features can be provided to the assistive device (102) via an update when the firmware update becomes available. This enables system (100) to continually improve features and device capability. As patient/user needs change through their patient journey, software features can be enabled or disabled to meet the needs of the patient, e.g., as a patient transitions from a lower mobility classification (K2) to higher classifications (K3) or vice versa, different, more appropriate firmware can be installed on the assistive device (102) as appropriate for their functional needs and activities. Security and bug fixes can be applied via maintenance updates in the field, allowing system (100) to meet post-market surveillance regulatory requirements, reducing the need for the assistive device (102) to be returned to a central location for updates, reducing patient and clinic inconvenience and appointments, simultaneously reducing/negating the need for loaners.

In an embodiment, the wireless connection link established between the mobile device (103) and the assistive device (102) is secured using encryption. Specifically, the connection is encrypted with a unique encryption key that is exclusively associated with the assistive device (102). This unique encryption key ensures that data exchanged over the wireless connection remains confidential and protected from unauthorized access, thereby enhancing the security and integrity of the communication between the mobile device (103) and the assistive device (102).

The system (100) supports remote monitoring and collaboration through a web-based portal accessible to healthcare providers. Remote users can access patient data, review gait analysis reports, and provide treatment recommendations. This feature is particularly valuable for telemedicine applications and the management of patients with mobility impairments. By combining wearable technology, advanced data processing, and cloud computing, the proposed system (100) offers a powerful tool for understanding and improving human gait. Its potential applications extend to various fields, including prosthetics, orthotics, rehabilitation, and sports performance analysis. Ensuring the security and privacy of patient data is paramount in remote monitoring systems. Robust encryption protocols are implemented to protect data both in transit and at rest. Access to patient data is strictly controlled, with appropriate role-based access permissions granted to healthcare providers and authorized personnel. Additionally, the system (100) adheres to relevant data protection regulations and industry standards to safeguard patient confidentiality.

To maximize the effectiveness of remote monitoring, engaging patients in the process is crucial. Clear instructions and training on how to use the system (100) are provided. User-friendly interfaces and regular feedback mechanisms can enhance patient satisfaction and adherence. Gamification elements or progress tracking features can also motivate patients to actively participate in data collection. Incorporating patient-reported outcomes into the system (100) provides valuable qualitative data that complements objective measurements. The patient-reported outcomes can capture information about pain, function, and quality of life, offering a comprehensive perspective on the patient's experience. By correlating the patient-reported outcomes with measurement data and video analysis, healthcare providers can gain deeper insights into treatment efficacy and patient outcomes. The system (100) employs advanced analytics to extract actionable insights from the collected data. Machine learning and generative AI mechanisms are employed to identify trends, patterns, and anomalies, enabling early detection of potential issues and facilitating timely interventions. Furthermore, clinical decision support tools can be integrated into the system (100) to provide data-driven recommendations, aiding healthcare providers in making informed and precise decisions.

The foregoing description of the specific embodiments will so fully reveal the general nature of the embodiments herein that others can, by applying current knowledge, readily modify and/or adapt for various applications such specific embodiments without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation. Therefore, while the embodiments herein have been described in terms of preferred embodiments, those skilled in the art will recognize that the embodiments herein can be practiced with modification within the scope of the embodiments as described herein.

## Claims

1. A mobile device (103) for monitoring and visualizing a gait of a user (101) (101), comprising:
a camera (204);
I/O interface (202);
a memory (203) comprising an application (104); and
a processor (201) connected to the memory (203), wherein the processor (201) is configured to:
display a user interface (401) via the application (104), wherein the user interface (401) allows the user (101) to connect to an assistive device (102) worn by the user (101);
establish a wireless connection with the assistive device (102) via a short-range communication protocol, wherein the assistive device (102) comprising a plurality of sensors produces measurement data indicating movements of the user (101);
start a gait session to receive the measurement data from the plurality of sensors upon successful establishment of the wireless connection link, wherein the gait session initializes measurement data transmission from the plurality of sensors;
receive an initialization signal from the assistive device (102) confirming readiness of the plurality of sensors to transmit the measurement data;
activate the camera (204) to record a video data of the user (101) wearing the assistive device (102) concurrently after receiving the initialization signal, wherein the video data is recorded while receiving the measurement data from the plurality of sensors during the gait session;
apply timestamps to frames of the video data of the user (101) with respect to the measurement data based on the initialization signal to obtain time-synchronized gait visualization data (301); and
store the time-synchronized gait visualization data (301) within the application (104) for subsequent retrieval and analysis.

2. The mobile device (103) as claimed in claim 1, wherein the processor (201) is configured to:
playback the gait session comprising the time-synchronized gait visualization data (301) through the user interface (401) on the mobile device (103), wherein the user interface (401) comprises the video data and a graphical chart indicating the gait of the user (101);
receive a user input to navigate to different timestamps within the gait session for analyzing video frames and associated timestamped measurement data; and
allow navigation to the different timestamps within the gait session based on the user input.

3. The mobile device (103) as claimed in claim 1 or 2, wherein the processor (201) is configured to:
upload the gait session comprising the time-synchronized gait visualization data (301) to a remote server (105) for retrieval and analysis at a later date or by a different user.

4. The mobile device (103) as claimed in any preceding claim, wherein applying timestamps to frames of the video data of the user (101) with respect to the measurement data comprises:
send an initial timestamp to the assistive device (102) via the short-range communication protocol while recording the video data of the user (101) wearing the assistive device (102);
receive a response comprising a current time marking start of the measurement data recording from the assistive device (102), wherein the assistive device (102) initializes recording of the measurement data using the plurality of sensors at the time of sending the response;
determine a delta time representing a time difference between the initial timestamp and the current time marking start of the measurement data recording; and
apply timestamps to frames of the video data of the user (101) with respect to the measurement data based on the delta time, opionally
wherein the video data is trimmed to match the start time of recording of the measurement data by the plurality of sensors based on the delta time.

5. The mobile device (103) as claimed in any preceding claim, wherein applying timestamps to frames of the video data of the user (101) with respect to the measurement data comprises:
detecting a unique sound generated and played by the assistive device (102) at moment of initiating the plurality of sensors for recording the measurement data, wherein the unique sound is detected while recording the video data; and
applying timestamps to frames of the video data of the user (101) by trimming the video data to align at least one frame of the video data corresponding to the detected unique sound.

6. The mobile device (103) as claimed in any preceding claim, wherein the processor (201) is configured to;
display the user interface (401) to configure an activity session to be performed by the user (101) wearing the assistive device (102), wherein the user interface (401) comprises a plurality of activity parameters to be configured for the activity session to be performed by the user (101) wearing the assistive device (102);
perform the activity session to be performed by the user (101) wearing the assistive device (102) based on an input received from the user corresponding to the plurality of activity parameters, optionally
wherein the plurality of activity parameters comprises at least one of a session name, a session type, and a feedback survey.

7. The mobile device (103) as claimed in any preceding claim, wherein the user interface (401) displays a plurality of user details corresponding to the gait sessions of the user (101), wherein the plurality of user details comprises at least one of a battery status of the assistive device (102), data synchronization status of the gait sessions, current mode indicating physical status of the user (101) and current status of the assistive device (102), and number of steps taken by the user (101) for a period of time.

8. The mobile device (103) as claimed in any preceding claim, wherein the processor (201) is configured to:
send a firmware update request message of the assistive device (102) to the remote server (105);
receive a firmware update response message from the remote server (105), wherein the firmware update response message comprises a firmware to be updated at the assistive device (102); and
transmit the firmware to the assistive device (102) over the short-range communication protocol.

9. The mobile device (103) as claimed in any preceding claim, wherein the wireless connection between the mobile device (103) and the assistive device (102) is encrypted with a unique encryption key specific to the assistive device (102).

10. A method for monitoring and visualizing a gait of a user (101) (101) comprises:
displaying, by a mobile device (103), a user interface (401) via an application (104) stored in the mobile device (103), wherein the user interface (401) allows the user (101) to connect to an assistive device (102) worn by the user (101);
establishing, by the mobile device (103), a wireless connection with the assistive device (102) via a short-range communication protocol, wherein the assistive device (102) comprising a plurality of sensors produces a measurement data indicating movements of the user (101);
starting, by the mobile device (103), a gait session to receive the measurement data from the plurality of sensors upon successful establishment of the wireless connection link, wherein the gait session initializes measurement data transmission from the plurality of sensors;
receiving, by the mobile device (103), an initialization signal from the assistive device (102) confirming readiness of the plurality of sensors to transmit the measurement data;
activating, by the mobile device (103), a camera (204) of the mobile device (103) to record a video data of the user (101) wearing the assistive device (102) concurrently after receiving the initialization signal, wherein the video data is recorded while receiving the measurement data from the plurality of sensors during the gait session;
applying, by the mobile device (103), timestamps to frames of the video data of the user (101) with respect to the measurement data based on the initialization signal to obtain time-synchronized gait visualization data (301); and
storing, by the mobile device (103), the time-synchronized gait visualization data (301) within the application (104) for subsequent retrieval and analysis.

11. The method as claimed in claim 10, the method comprises:
receiving, by the mobile device (103), a user input to navigate to different timestamps within the gait session for analyzing video frames and associated timestamped sensor data, wherein the mobile device (103) is configured to playback the gait session comprising the time-synchronized gait visualization data (301) through the user interface (401) on the mobile device (103), wherein the user interface (401) comprises the video data and a graphical chart indicating the gait of the user (101); and
allowing, by the mobile device (103), navigation to the different timestamps within the gait session based on the user input.

12. The method as claimed in claim 10 or 11, the method comprises transmitting by the mobile device (103), the gait session comprising the time-synchronized gait visualization data (301) to a remote server (105) for retrieval and analysis at a later date or by a different user.

13. The method as claimed in claim 10 to 12, wherein applying, by the mobile device (103), timestamps to frames of the video data of the user (101) with respect to the measurement data comprises:
sending, by the mobile device (103), an initial timestamp to the assistive device (102) via the short-range communication protocol while recording the video data of the user (101) wearing the assistive device (102);
receiving, by the mobile device (103), a response comprising a current time marking start of the measurement data recording from the assistive device (102), wherein the response comprising an internal clock time of the assistive device (102), wherein the assistive device (102) initializes recording of the measurement data using the plurality of sensors at the time of sending the response;
determining, by the mobile device (103), a delta time representing a time difference between the initial timestamp and the current time marking start of the measurement data recording; and
applying, by the mobile device (103), timestamps to frames of the video data of the user (101) with respect to the measurement data based on the delta time, optionally wherein the video data is trimmed to match the start time of recording of the measurement data by the plurality of sensors based on the delta time.

14. The method as claimed in claim 10 to 13, wherein applying, by the mobile device (103), timestamps to frames of the video data of the user (101) with respect to the measurement data comprises:
detecting, by the mobile device (103), a unique sound generated and played by the assistive device (102) at moment of initiating the plurality of sensors for recording the measurement data, wherein the unique sound is detected while recording the video data; and
applying, by the mobile device (103), the timestamps to the frames of the video data of the user (101) by trimming the video data to align at least one frame of the video data corresponding to the detected unique sound.

15. The method as claimed in any one of claims 10 to 14, the method comprises:
sending, by the mobile device (103), a firmware update request message of the assistive device (102) to the remote server (105);
receiving by the mobile device (103), a firmware update response message from the remote server (105), wherein the firmware update response message comprises a firmware to be updated at the assistive device (102); and
transmitting, by the mobile device (103), the firmware to the assistive device (102) over the short-range communication protocol.
